# EUROPÄISCHE PATENTSCHRIFT

(11) **EP 0 548 590 B1**
(45) Veröffentlichungstag und Bekanntmachung des Hinweises auf die Patenterteilung: **18.10.1995**
(21) Anmeldenummer: 92120373.3
(22) Anmeldetag: 28.11.1992
(51) Int. Cl.: C07C 233/43, C07C 271/28

(54) **N4-substituierte 1-Alkoxy-2-acylamino-4-aminobenzole, Verfahren zu ihrer Herstellung und ihre Verwendung**
N4-substituted 1-alkoxy-2-acylamino-4-aminobenzenes, method for their preparation and use
1-Alcoxy-2-acylamino-4-aminobenzènes, procédés de leur préparation et leur utilisation

(30) Priorität: 20.12.1991 DE 4142132
(43) Veröffentlichungstag der Anmeldung: 30.06.1993
(73) Patentinhaber: HOECHST AKTIENGESELLSCHAFT, 65926 Frankfurt am Main (DE)
(72) Erfinder: Steckelberg, Willi, Dr., W-6238 Hofheim/Ts (DE); Koch, Peter, Dr., W-6053 Obertshausen (DE)

(56) Entgegenhaltungen:
- DE-A- 3 237 004
- FR-A- 2 652 350
- GB-A- 2 232 419

## Beschreibung

Die vorliegende Erfindung betrifft Verbindungen der allgemeinen Formel I 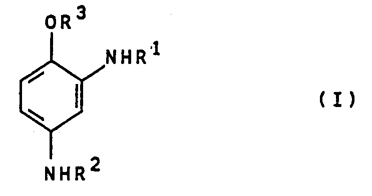
worin
- R¹: Formyl oder (C₁-C₃)-Alkylcarbonyl,
- R²: die Gruppe CH₂-CH(A)-B, worin A für Hydroxy und B für Wasserstoff oder Methyl oder A für Wasserstoff und B für Hydroxymethyl stehen oder die Gruppe COO-CH(X)-CH₂-D, worin X für Wasserstoff oder Methyl und D für Chlor oder Brom oder X für Wasserstoff und D für Chlormethyl oder Brommethyl stehen, und
- R³: (C₁-C₄)-Alkyl
bedeuten.

Unter (C₁-C₄)-Alkyl werden Methyl, Ethyl, n-Propyl, i-Propyl, n-Butyl, sek-Butyl, i-Butyl und tert-Butyl verstanden. Analoges gilt für (C₁-C₃)-Alkylcarbonyl. R¹ bedeutet bevorzugt Formyl, Acetyl und Propionyl. R² bedeutet bevorzugt Hydroxyethyl oder COOCH₂CH₂Cl. R³ bedeutet bevorzugt Methyl.
Bevorzugte Verbindungen der allgemeinen Formel I sind β-Chlorethyl-N-(3-acetylamino-4-methoxyphenyl)carba-mat und 2-Acetylamino-4-(β-hydroxyethylamino)-1-methoxybenzol
Die Verbindungen der allgemeinen Formel I können dadurch hergestellt werden, daß eine Verbindung der allgemeinen Formel II 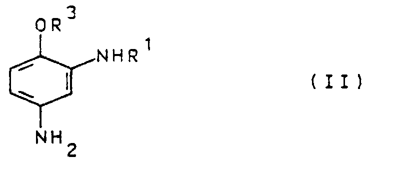
mit einer Verbindung der allgemeinen Formel III

ClCOOCH(X)-CH₂-D (III)

zu einer Verbindung der allgemeinen Formel Ia 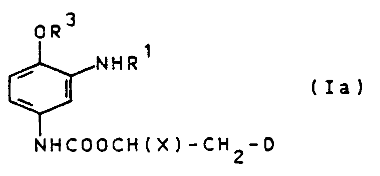
umgesetzt wird und diese gegebenenfalls in eine Verbindung der allgemeinen Formel Ib 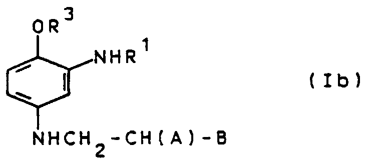
überführt wird, wobei R¹, R³, A, B, D und X wie oben angegeben definiert sind.

Die Umsetzung der Verbindungen der allgemeinen Formel II mit denen der allgemeinen Formel III geschieht bevorzugt derart, daß die Verbindung der allgemeinen Formel II in einem inerten Lösungsmittel vorgelegt, auf eine Temperatur zwischen Raumtemperatur und Rückflußtemperatur, besonders bevorzugt zwischen 60°C und Rückflußtemperatur, erhitzt und dann die Verbindung der allgemeinen Formel III in äquimolarer Menge oder einem geringen Überschuß zudosiert wird. Geeignete inerte Lösungsmittel sind beispielsweise Dioxan, Tetrahydrofuran, Ethylenglykoldimethylether oder niedere Alkohole wie Methanol, Ethanol oder Propanol. Die genannten Lösungsmittel können auch im Gemisch mit Wasser eingesetzt werden.

Es ist besonders bevorzugt, die Reaktion unter Verwendung einer Hilfsbase auszuführen, die mit vorgelegt oder kontinuierlich zugegeben werden kann. Geeignete Hilfsbasen sind beispielsweise Alkalihydroxide, Erdalkalioxide, (Erd)alkalicarbonate oder -hydrogencarbonate sowie tertiäre Amine. Bevorzugt sind Alkali- und Erdalkalicarbonate, wie Natrium-, Kalium- oder Calciumcarbonat.

Die so erhaltenen erfindungsgemäßen Verbindungen der allgemeinen Formel Ia können gegebenenfalls in erfindungsgemäße Verbindungen der allgemeinen Formel Ib überführt werden. Dies geschieht insbesondere durch Behandlung mit einer Base.

Bevorzugt werden dazu die Verbindungen der allgemeinen Formel Ia in Wasser, einem organischen Lösungsmittel oder Mischungen davon vorgelegt, bei Raumtemperatur eine Lauge in geringem Überschuß zugefügt und bei Temperaturen zwischen 10 und 60 °C, bevorzugt zwischen Raumtemperatur und 40 °C, gerührt bis die Bildung des intermediär auftretenden Oxazolidinons (siehe DE-A 39 31 836) vollständig ist. Anschließend wird bei Temperaturen zwischen 40 °C und Rückflußtemperatur weitergerührt bis die Reaktion beendet ist. Gewöhnlich werden für die gesamte Reaktion 8 bis 12 Stunden benötigt.

Geeignete organische Lösungsmittel sind beispielsweise niedere Alkohole wie Methanol, Ethanol oder Propanol sowie wassermischbare Ether, wie Ethylenglykoldimethylether, Dioxan oder Tetrahydrofuran.

Bevorzugte Lösungsmittel sind Methanol, Ethanol und Ethylenglykoldimethylether.

Geeignete Laugen sind Natronlauge und Kalilauge.

Die Ausgangsverbindungen der allgemeinen Formel II können in einfacher und an sich bekannter Weise durch Reduktion der entsprechenden Nitroverbindungen der allgemeinen Formel IV 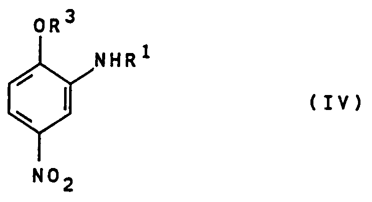
worin R¹ und R³ wie oben angegeben definiert sind, erhalten werden.
Beispielsweise geschieht die Reduktion mit niedervalenten oder unedlen Metallen oder katalytisch (siehe z. B. EP-A 250 099, J. Am. Chem. Soc. 69 (1947) 583). Bevorzugt ist die katalytische Hydrierung mit üblichen Katalysatoren wie Palladium auf Aktivkohle, Platin, Platindioxid oder Raney-Nickel. Geeignete Lösungsmittel sind beispielsweise Wasser, niedere Alkohole wie Methanol, Ethanol oder Propanol, Toluol, Eisessig, Essigester oder Ether-Verbindungen wie Ethylenglykoldimethylether, Dioxan oder Tetrahydrofuran.

Die Reaktionstemperatur liegt vorteilhafterweise zwischen Raumtemperatur und 120°C, bevorzugt zwischen 40 und 80°C.

Es kann bei Normaldruck oder einem Überdruck von bis zu 40 bar gearbeitet verden. Bevorzugt sind 10 bis 30 bar. Die so erhaltenen Verbindungen der allgemeinen Formel II können nach Abtrennung des Katalysators in üblicher Weise isoliert und weiterverarbeitet werden. Es ist jedoch bevorzugt, sie ohne Isolierung direkt mit den Verbindungen der allgemeinen Formel III umzusetzen. Lösungsmittel für diese Umsetzung ist somit bevorzugt das Filtrat der katalytischen Hydrierung der Vorstufe.

Die Verbindungen der allgemeinen Formel IV können in einfacher und an sich bekannter Weise durch Umsetzung eines 2-Amino-1-alkoxy-4-nitrobenzols mit Ameisensäure (J. Am. Chem. Soc. 103 (1981) 5599) oder einem Carbonsäureanhydrid (EP-A 250 099; Rec. Trav. Chim. Pays-Bas 25 (1906) 18; J. Org. Chem. 17 (1952) 1216) erhalten werden. Die Temperaturen dieser Umsetzungen liegen vorteilhafterweise bei 20 bis 100°C, vorzugsweise bei 50 bis 90°C.

Überraschend wurde gefunden, daß bei Verwendung von verdünnter Carbonsäure als Solvens, d. h. von 30 bis 90 %iger, bevorzugt 40 bis 60 %iger Carbonsäure, ohne Ausbeuteverlust ein Reinigungseffekt der resultierenden Verbindung der allgemeinen Formel IV erzielt wird. Es ist somit möglich, 2-Amino-1-alkoxy-4-nitrobenzole technischer Qualität mit isomeren Verunreinigungen bis zu 5 Gew% einzusetzen. Nach Aufarbeitung in üblicher Weise resultiert ein isomerenreines Produkt, die Verunreinigungen verbleiben in der Mutterlauge.

Die Verbindungen der allgemeinen Formel I können zur Herstellung von Verbindungen der allgemeinen Formel V 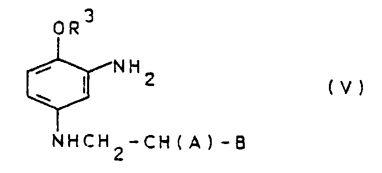
worin R³, A und B wie oben angegeben definiert sind, oder ihrer Säureadditionssalze verwendet werden.

Die Verbindungen der allgemeinen Formel V bzw. ihre Säureadditionssalze sind Farbkuppler in Oxidationsfarbstoffen und spielen insbesondere eine bedeutende Rolle für die Färbung von Haaren und Pelzen (siehe beispielsweise DE-A 31 32 885, DE-A 29 51 377, DE-A 36 25 916, DE-A 35 45 371, DE-A 34 41 148, DE-A 36 09 504 und DE-A 38 44 517).

Die Säureadditionssalze der Verbindungen der allgemeinen Formel V können mit anorganischen oder organischen Säuren gebildet werden, wobei insbesondere Chlorwasserstoff oder Schwefelsäure bevorzugt sind. Die Säureadditionssalze können in bekannter Weise durch Vereinigung der Komponenten, zweckmäßigerweise in einem geeigneten Lösungs- oder Verdünnungsmittel, hergestellt werden. Allerdings fallen bei der Synthese die Verbindungen der allgemeinen Formel V normalerweise direkt in Form ihrer Säureadditionssalze an.

Erfindungsgemäß können die Verbindungen der allgemeinen Formel Ib direkt in Verbindungen der allgemeinen Formel V überführt werden, während Verbindungen der allgemeinen Formel Ia erst wie oben angegeben zu solchen der allgemeinen Formel Ib umgesetzt werden müssen. Es ist in diesem Fall aber nicht nötig, die Verbindung der allgemeinen Formel Ib zu isolieren. Vielmehr kann sich die Entacylierung direkt an ihre Synthese anschließen. Die Entacylierung der Verbindungen der allgemeinen Formel Ib geschieht in an sich bekannter Weise durch Erhitzen mit einer starken Säure (siehe beispielsweise Beilsteins Handbuch der Organischen Chemie, Band 13, III. Ergänzungswerk, Seite 1201, Springer-Verlag). Geeignete Säuren sind insbesondere Schwefelsäure, Salzsäure und Phosphorsäure.

Zweckmäßigerweise wird die Verbindung der allgemeinen Formel Ib in Wasser, einem Alkohol mit 1 bis 4 Kohlenstoffatomen, einem wassermischbaren Ether, wie Ethylendiglykoldimethylether oder Mischungen der genannten Lösungsmittel untereinander vorgelegt und mit einem Überschuß der starken Säure mehrere Stunden unter Rückfluß erhitzt.

Durch die Bereitstellung der erfindungsgemäßen Verbindungen der allgemeinen Formel I steht somit auch ein vorteilhaftes Verfahren zur Herstellung der Verbindungen der allgemeinen Formel V, ausgehend von 2-Amino-1-alkoxy-4-nitrobenzolen, zur Verfügung.

Beispielsweise kann 2-Amino-4-hydroxy-ethylamino-1-methoxybenzol ausgehend von käuflichem 2-Amino-4-nitro-1-methoxybenzol über die erfindungsgemäßen Verbindungen β-Chlorethyl-N-(3-acetylamino-4-methoxyphenyl)carbamat und 2-Acetylamino-4-(β-hydroxyethylamino)-1-methoxybenzol hergestellt werden.

### Beispiel 1

### Herstellung von β-Chlorethyl-N-(3-acetylamino-4-methoxyphenyl)carbamat

### a) 2-Acetylamino-4-nitro-1-methoxybenzol:

In 860 ml Essigsäure und 600 ml Wasser (oder dem vereinigten Haupt- und ersten Waschfiltrat eines vorhergehenden Ansatzes) werden 504 g (3 Mol) 2-Amino-4-nitro-1-methoxybenzol eingetragen und auf 80 °C erwärmt. Innerhalb von 10 Minuten werden 358 g (3,2 Mol) Acetanhydrid zugetropft. Es wird vier Stunden bei 80 °C nachgerührt und dann unter Rühren durch Entfernen des Heizbades auf Raumtemperatur und schließlich auf 15 °C abgekühlt. Die ausgefallenen hellgelben Nadeln werden abgesaugt, mit 300 ml 60 %iger Essigsäure und 1500 ml Wasser in mehreren Portionen gewaschen und getrocknet.
Ausbeute: 592,3 g (93,6 %)
Fp.: 174 - 175 °C
Nach chromatografischen Untersuchungen einheitlich.

### b) β-Chlorethyl-N-(3-acetylamino-4-methoxyphenyl)carbamat:

126 g (0,6 Mol) 2-Acetylamino-4-nitro-1-methoxybenzol werden mit 350 ml Ethylenglykoldimethylether (DME) in einen Edelstahlautoklaven überführt, mit 1,5 g Palladium-Kohle-Katalysator (10 %) versetzt und bei 60 °C bei einem Wasserstoffdruck von 20 bar katalytisch hydriert. Nach Aufnahme der berechneten Menge Wasserstoff wird auf Raumtemperatur abgekühlt, vom Katalysator abfiltirert, der Rückstand mit wenig DME gewaschen und das Filtrat mit 32,2 g (0,32 Mol) Calciumcarbonat versetzt und auf 78 °C erwärmt. Innerhalb von drei Stunden werden 90 g (0,63 Mol) Chlorameisensäurechlorethylester zugetropft, vier Stunden bei 78 °C nachgerührt, auf 30 °C abgekühlt und 500 ml Wasser sowie 500 ml Eiswasser zugegeben. Es wird abgesaugt, mit 500 ml Wasser gewaschen und getrocknet.
Ausbeute: 168,4 g (97,9 %)
Fp.: 176 - 177 °C
IR-Spektrum (KBr): ν = 1705 cm⁻¹ (C = O Carbamat), 1660 cm⁻¹ (C = O Amid), 1600 cm⁻¹ (C = C), 1240 cm⁻¹ (Ether).
¹H-NMR-Spektrum ([D₆]-DMSO): δ = 2,1 (s, 3H, -COCH₃), 3,8 (s, 3H, -OCH₃), 3,9 (t, 2H, OCH₂-CH₂Cl), 4,4 (t, 2H, -OCH₂-CH₂Cl), 7,0 (m, 1H, H ar.), 7,2 (m, 1H, H ar.), 8,0 (m, 1H, H ar.), 9,1 (br. s, 1H, NH), 9,5 (br. s, 1H, NH).

### Beispiel 2

### Herstellung von 2-Acetylamino-4-(β-hydroxyethylamino)-1-methoxybenzol

86 g (0,3 Mol) β-Chlorethyl-N-(3-acetylamino-4-methoxyphenyl)carbamat werden in 90 ml Ethanol und 300 ml Wasser suspendiert, mit 26,4 g (0,33 Mol) 50 % Natronlauge versetzt und fünf Stunden bei 30 °C gerührt. Es werden noch 75,2 g (0,66 Mol) Natronlauge zugegeben und fünf Stunden auf 60 °C erwärmt. Nach Abkühlen auf Raumtemperatur wird mit Salzsäure neutralisiert, mit 200 ml Wasser versetzt und das Produkt durch Absaugen und Waschen mit Wasser isoliert.
Ausbeute: 57,4 g (85,4 %)
Fp.: 82 - 83 °C
IR-Spektrum (KBr): ν = 3260 cm⁻¹ (OH/NH), 1660 cm⁻¹ (C = O Amid), 1600 cm⁻¹ (C = C), 1535 cm⁻¹ (Amid II), 1225 cm⁻¹ (Ether).
¹H-NMR-Spektrum ([D₆]-DMSO): δ = 2,1 (s, 3H, -COCH₃), 3,0 (dt, 2H, -CH₂-CH₂OH), 3,5 (dt, 2H, CH₂-CH₂OH), 3,7 (s, 3H, -OCH₃), 4,6 (t, 1H, -OH), 5,1 (t, 1H, -NH-C₂H₄OH), 6,3 (m, 1H, H ar.), 6,8 (m, 1H, H ar.), 7,4 (m, 1H, H ar.), 8,8 (br. s, 1H, NH-COCH₃).

### Beispiel 3

### Herstellung von 2-Amino-4-hydroxyethylamino-1-methoxybenzol

### a) aus β-Chlorethyl-N-(3-acetylamino-4-methoxyphenyl)carbamat

86 g (0,3 Mol) β-Chlorethyl-N-(3-acetylamino-4-methoxyphenyl)carbamat werden in 90 ml Ethanol und 300 ml Wasser suspendiert, mit 26,4 g (0,33 Mol) 50 % Natronlauge versetzt und fünf Stunden bei 30 °C gerührt. Es werden noch 75,2 g (0,66 Mol) Natronlauge zugegeben und fünf Stunden auf 60 °C erwärmt. Der Ansatz wird jetzt mit 85 g (0,7 Mol) 30 % Salzsäure versetzt und fünf Stunden unter Rückfluß erhitzt. Schließlich wird mit Natronlauge auf pH 8 gestellt, auf 10 °C gekühlt und das auskristallisierte Produkt abgesaugt.
Ausbeute: 44,7 g (82 %)
Fp.: 99 - 100 °C
IR-Spektrum (KBr): ν = 3300 cm⁻¹ (OH/NH), 1600 cm⁻¹ (C = C), 1225 cm⁻¹ (Ether), 1055/1030 cm⁻¹ (CH₂OH). ¹H-NMR-Spektrum ([D₆]-DMSO): δ = 2,9 (dt, 2H, -CH₂-CH₂-OH), 3,5 (dt, 2H, -CH₂-CH₂OH), 3,6 (s, 3H, -OCH₃), 4,5 (br. s, 2H, -NH₂), 4,6 (t, 1H, -OH), 4,7 (t, 1H, -NH), 5,8 (m, 1H, H ar.), 6,0 (m, 1H, H ar.), 6,6 (m, 1H, H ar.).

### b) aus 2-Acetylamino-4-(β-hydroxyethylamino)-1-methoxybenzol

22,4 g (0,1 Mol) 2-Acetylamino-4-(β-hydroxyethylamino)-1-methoxybenzol werden in 60 ml Wasser und 12,2 g (0,2 Mol) 30 %ige Salzsäure 5 Stunden unter Rückfluß erhitzt. Man stellt mit Natronlauge auf pH 8, kühlt auf 10°C und saugt das auskristallisierte Produkt ab. Es wird mit Wasser gewaschen und getrocknet.
Ausbeute: 16,4 g (90 %)
physikalische Daten wie unter a)

## Patentansprüche

1. Verbindungen der allgemeinen Formel I 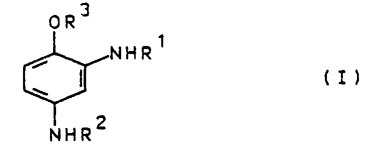 worin
R¹ Formyl oder (C₁-C₃)-Alkylcarbonyl,
R² die Gruppe CH₂-CH(A)-B, worin A für Hydroxy und B für Wasserstoff oder Methyl oder A für Wasserstoff und B für Hydroxymethyl stehen oder die Gruppe COO-CH(X)-CH₂-D, worin X für Wasserstoff oder Methyl und D für Chlor oder Brom oder X für Wasserstoff und D für Chlormethyl oder Brommethyl stehen, und
R³ (C₁-C₄)-Alkyl
bedeuten.

2. Verbindungen gemäß Anspruch 1, dadurch gekennzeichnet, daß R¹ Formyl, Acetyl oder Propionyl bedeutet.

3. Verbindungen gemäß Anspruch 1 und/oder 2, dadurch gekennzeichnet, daß R² Hydroxyethyl oder COOCH₂CH₂Cl bedeutet.

4. Verbindungen gemäß einem oder mehreren der Ansprüche 1 bis 3, dadurch gekennzeichnet, daß R³ Methyl bedeutet.

5. β-Chlorethyl-N-(3-acetylamino-4-methoxyphenyl)carbamat.

6. 2-Acetylamino-4-(β-hydroxyethylamino)-1-methoxybenzol.

7. Verfahren zur Herstellung von Verbindungen der allgemeinen Formel I gemäß einem oder mehreren der Ansprüche 1 bis 6, dadurch gekennzeichnet, daß eine Verbindung der allgemeinen Formel II 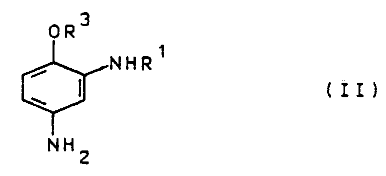 mit einer Verbindung der allgemeinen Formel III
ClCOOCH(X)-CH₂-D (III)
zu einer Verbindung der allgemeinen Formel Ia 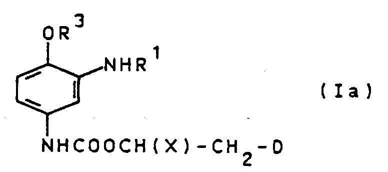 umgesetzt wird und diese gegebenenfalls in eine Verbindung der allgemeinen Formel Ib 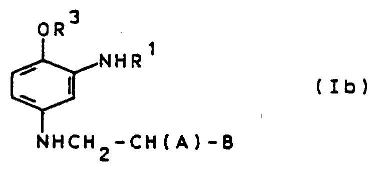 überführt wird, wobei R¹, R³, A, B, D und X wie in Anspruch 1 angegeben definiert sind.

8. Verwendung der Verbindungen gemäß einem oder mehreren der Ansprüche 1 bis 6 der allgemeinen Formel I zur Herstellung von Verbindungen der allgemeinen Formel V 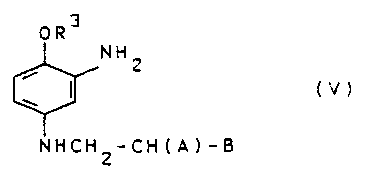 worin R³, A und B wie in Anspruch 1 angegeben definiert sind, oder ihrer Säureadditionssalze.

## Claims

1. Compounds of the general formula I 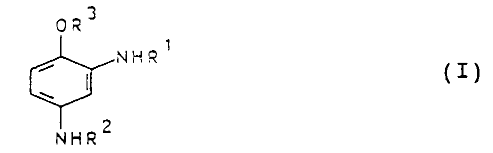 wherein
R¹ denotes formyl or (C₁-C₃)-alkylcarbonyl,
R² denotes the group CH₂-CH(A)-B, wherein A represents hydroxyl and B represents hydrogen or methyl, or A represents hydrogen and B represents hydroxymethyl, or the group COO-CH(X)-CH₂-D, wherein X represents hydrogen or methyl and D represents chlorine or bromine, or X represents hydrogen and D represents chloromethyl or bromomethyl, and
R³ denotes (C₁-C₄)-alkyl.

2. Compounds according to Claim 1, characterised in that R¹ denotes formyl, acetyl or propionyl.

3. Compounds according to Claim 1 and/or 2, characterised in that R² denotes hydroxyethyl or COOCH₂CH₂Cl.

4. Compounds according to one or more of Claims 1 to 3, characterised in that R³ denotes methyl.

5. β-Chloroethyl N-(3-acetylamino-4-methoxyphenyl)carbamate.

6. 2-Acetylamino-4-(β-hydroxyethylamino)-1-methoxybenzene.

7. Process for the preparation of compounds of the general formula I according to one or more of Claims 1 to 6, characterised in that a compound of the general formula II 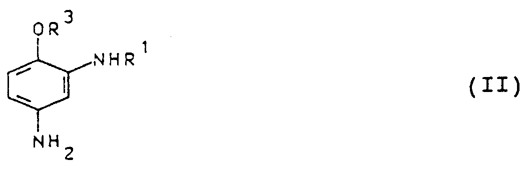 is reacted with a compound of the general formula III
ClCOOCH(X)-CH₂-D (III)
to give a compound of the general formula Ia 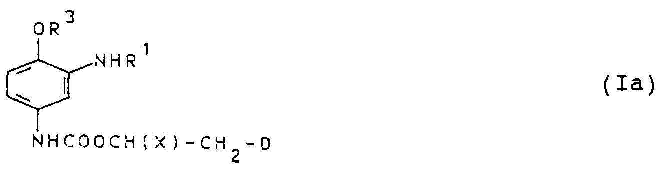 and, if appropriate, this is converted into a compound of the general formula Ib 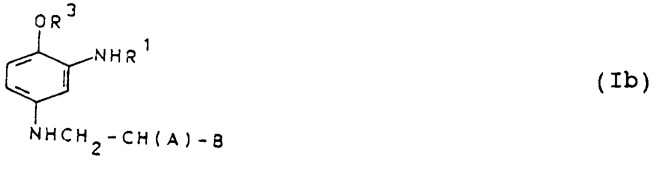 wherein R¹, R³, A, B, D and X are defined as stated in Claim 1.

8. Use of the compounds according to one or more of Claims 1 to 6 of the general formula I for the preparation of compounds of the general formula V 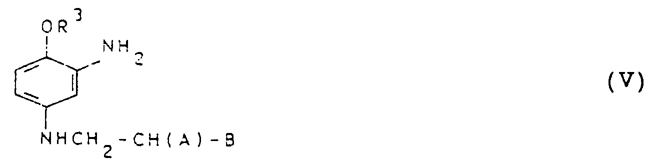 wherein R³, A and B are defined as stated in Claim 1, or their acid addition salts.

## Revendications

1. Composés de formule générale I 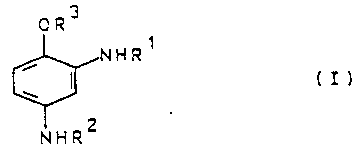 dans laquelle
R¹ est le radical formyle ou un radical (alkyle en C₁-C₃)carbonyle,
R² est le groupe CH₂-CH(A)-B, dans lequel A est un groupe hydroxy et B est un hydrogène ou le radical méthyle, ou A est un hydrogène et B est le radical hydroxyméthyle, ou bien le groupe COO-CH(X)-CH₂-D, dans lequel X est un hydrogène ou le radical méthyle et D est le chlore ou le brome, ou encore X est un hydrogène et D est le radical chlorométhyle ou bromométhyle, et
R³ est un radical alkyle en C₁-C₄.

2. Composés selon la revendication 1, caractérisés en ce que R¹ est le radical formyle, acétyle ou propionyle.

3. Composés selon la revendication 1 et/ou 2, caractérisés en ce que R² est le radical hydroxyéthyle ou COOCH₂CH₂Cl.

4. Composés selon l'une ou plusieurs des revendications 1 à 3, caractérisés en ce que R³ est le radical méthyle.

5. N-(3-acétylamino-4-méthoxyphényl)carbamate de β-chloroéthyle.

6. 2-acétylamino-4-(β-hydroxyéthylamino)-1-méthoxybenzène.

7. Procédé pour préparer des composés de formule générale I selon l'une ou plusieurs des revendications 1 à 6, caractérisé en ce qu'on fait réagir un composé de formule générale II 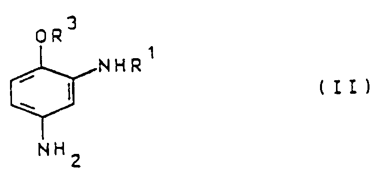 avec un composé de formule générale III
ClCOOCH(X)-CH₂-D (III)
pour obtenir un composé de formule générale Ia 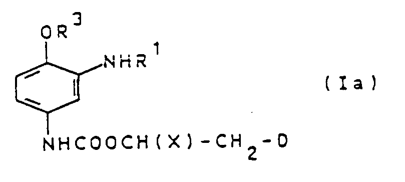 et on convertit éventuellement ce dernier en un composé de formule générale Ib 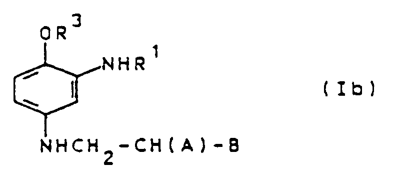 où R¹, R³, A, B, D et X ont les significations données dans la revendication 1.

8. Utilisation des composés selon l'une ou plusieurs des revendications 1 à 6 de formule générale I pour préparer des composés de formule générale V 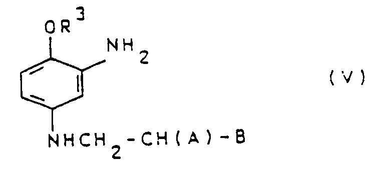 dans laquelle R³, A et B ont les significations données dans la revendication 1, ou de leurs sels d'addition avec un acide.
